# EUROPEAN PATENT SPECIFICATION

(11) **EP 1 438 076 B1**
(45) Date of publication and mention of the grant of the patent: **14.06.2006**
(21) Application number: 02789260.3
(22) Date of filing: 22.10.2002
(51) Int. Cl.: A61K 51/00, A61P 35/00, C07F 9/6524, C07F 9/6561

(54) **RADIOPHARMACEUTICAL AGENT FOR THE TREATMENT OF EARLY STAGE CANCER**
RADIOPHARMAZEUTISHES MITTEL ZUR BEHANDLUNG VON KREBS IM FRÜHSTADIUM
AGENT RADIOPHARMACEUTIQUE POUR LE TRAITEMENT DU CANCER A UN STADE PRECOCE

(30) Priority: 22.10.2001 US 355598 P
(43) Date of publication of application: 21.07.2004
(73) Proprietor: DOW GLOBAL TECHNOLOGIES INC., Midland, Michigan 48674 (US)
(72) Inventor: KIEFER, Garry, E., Lake Jackson, TX 77566 (US)
(74) Representative: Weickmann, Heinrich
(86) International application number: PCT/US2002/033918
(87) International publication number: WO 2003/035114

(56) References cited:
- WO-A-93/11801
- WO-A-94/03464
- WO-A-94/26753

## Description

This invention concerns the use of metal-ligand complexes for the manufacture of medicaments for treating cancer. More particularly, this invention concerns the manufacture of medicaments for treating epithelial cancer.

Metal ligand complexes are routinely used for medicinal applications. When radioactive metal ions are used, diagnostic imaging or therapy can be the end objective. Thus ^{99m}Tc, a pure gamma emitter, in the form of a metal ligand complex is routinely used as a diagnostic agent. In some cases, such as the use of ^{99m}TC-DTPA, injection of the complex into the bloodstream does not result in the radionuclide localizing in any tissue. Instead, the radionuclide is eliminated from the body by the kidneys into the urine. In other cases, the radionuclide does localize in desired specific organs or tissues.

The specific delivery of metals to soft tissue (that is, non-calcific) tumors has been an objective for scientists. Anghilery in Nuklearmedizin 23, 9-14 (1984) describes the difficulty in achieving this objective when he states that "there are no fundamental qualitative differences in the structural, biochemical and functional characteristics of a tumor compared to the normal cell." With the advent of monoclonal antibodies, a plethora of activity has emerged using these proteins to deliver radionuclides to soft tissue tumors [for example, A. R. Fritzberg et al., Pharm. Res. 5(6), 325 (1988)]. Bifunctional chelating agents were developed to bind the metal ions to the monoclonal antibody through a chelating agent (which metal-ligand- antibody system is termed a "conjugate") and many such conjugates have emerged. Currently, the covalent attachment of a small molecule to a large protein or antibody (referred to as "bifunctional") is receiving much attention as the method of choice for achieving tissue specificity. Some conjugates use gamma emitters such as ^{99m}Tc or ¹¹¹In for imaging (see, for example, U.S. Patents 4,454,106, 3,994,966, 4,662,420 and 4,479,930); and other proposed conjugates with particle emitters such as ⁶⁷Cu [see, for example, J. C. Roberts et al., Appl.Rad.Isotopes 40 (9), 775 (1989)] or ⁹⁰Y [see, for example, J.Nucl.Med. 26 (5), 503 (1985)] for therapy. It was believed that the use of the conjugates provided the answer to the site specific delivery of a metal ion to soft tissue tumors. However, in the practice of the use of these conjugates a series of problems has been observed. For example, the problems have involved the fragile nature of the antibody, the slow clearance of the radioactivity from the blood stream, the uptake of radioactivity in non-target tissues such as liver and kidney, and the potential of an immune response of the patient to the injected protein.

One such example of a bifunctional molecule is disclosed in Griffin, J.M.M. et al, "Simple, high yielding synthesis of trifunctional fluorescent lanthanide chelates", Tetrahedron Letters 42 (2001) pp. 1-3. Griffin discloses a lanthanide chelating ligand based on the cyclen (1, 4, 7, 10-tetraazacyclododecane) nucleus which possesses a single carboxyl group for conjugation to a biologically active species such as an antibody. However, this method is inherently complex and expensive since it requires the use of a specialized antibody in order to achieve tissue specificity.

Another approach to delivering metal ions to soft tissue cancers or tumors is by means of a metal ligand complex. Woolfenden et al. in Int. J. Nucl. Med. 10 (4), 251-256 (1983) found that ¹⁵³Sm-citrate and ¹⁵³Sm-chloride had a high liver uptake and suggested the use of higher stability chelates, such as ¹⁵³Sm-EDTA (ethylenediaminetetraacetic acid), could improve the tumor to liver ratio. More recently, J. Harvey Turner in Eur. J. Nucl. Med. 13, 432-438 (1987) studied ¹⁵³Sm chelates including HEDTA (hydroxyethylethylenediaminetriacetic acid). The ¹⁵³Sm-HEDTA chelates used a 20 to 1 HEDTA to Sm molar ratio. Tumor uptake was found to be significantly less' than that of ⁶⁷Ga-citrate; liver dose was much greater than tumor dose. He concluded that "it is unlikely that effective therapy doses of Sm-153 can be delivered to melanoma tumors by these and similar chelates." He suggested the use of monoclonal antibodies with ¹⁵³Sm.

Another attempt to have complexes deliver metal ions to soft tissue tumors was made by Tsc et al. in J. Nucl. Med. 30, 202-208 (1989) where they studied ¹⁵³Sm-EDTA at a 10 to 1 ligand to metal molar ratio. These researchers proved that the complex was stable and compared the use of high specific activity ¹⁵³Sm (1.7 Ci/mG) to low specific activity ¹⁵³Sm (1.1 mCi/mG) in mice bearing Lewis lung carcinoma. They proposed using the complex as an imaging agent using the high specific activity ¹⁵³Sm. However, these researchers also found significant uptake in the liver as shown by their biodistribution and images.

WO 94/03464 discloses polyazamacrocyclic compounds of formula {(CH₂)ₓ NR}_{y}, wherein x is 2, 3 or a combination of p2(s) and q3(s), where p+q = y and y is 3 or 4, and its use as a tissue specific chelate. The compound may be complexed with a metal to form a polyazamacrocyclic compound-metal complex.

Therefore, there is still a need for an adequate system to deliver radionuclides selectively to soft tissue tumors. Surprisingly, it has now been found that various tetraazamacrocyclic complexes give good soft tissue localization and can be used as therapeutic agents.

The present invention provides
a pharmaceutical formulation for the therapeutic treatment of a mammal having a disease state comprising:
(1) a radioactive chelate having a formula: 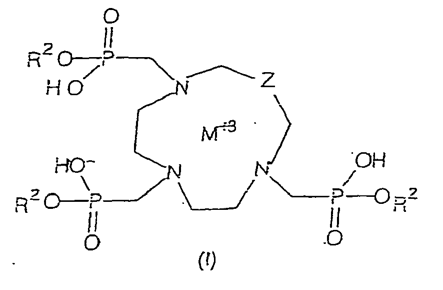 wherein Z is 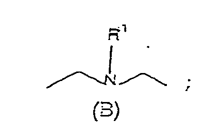 R¹ is 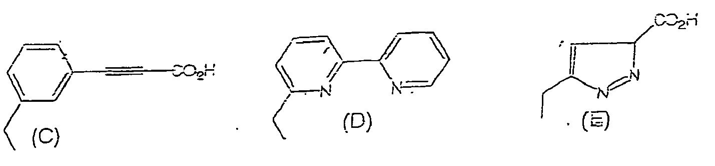 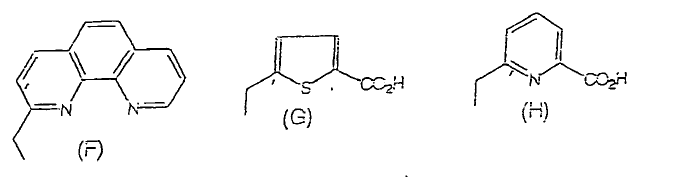 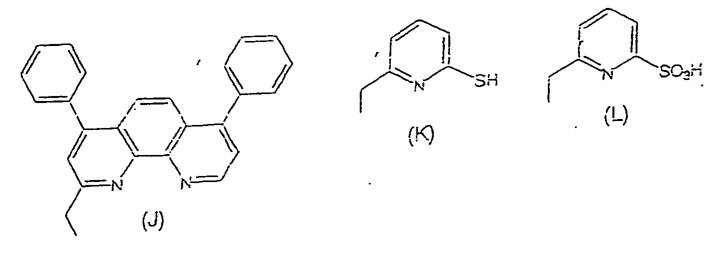 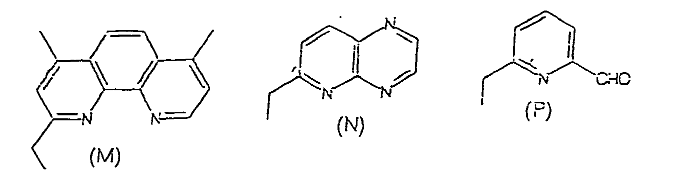 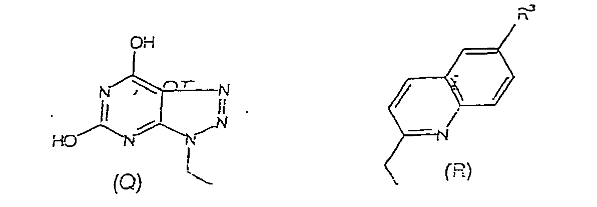 R² is methyl ethyl, propyl, butyl or H; and
   R³ is F, C1-C4 alkyl, O(C₁-C₄ alkyl) or Cl;
   M is a radioactive metal ion; or
   pharmaceutically salts thereof; and
(2) a pharmaceutically acceptable carrier.

The present invention also concerns the use of a formulation comprising
(1) a radioactive chelate having a formula: 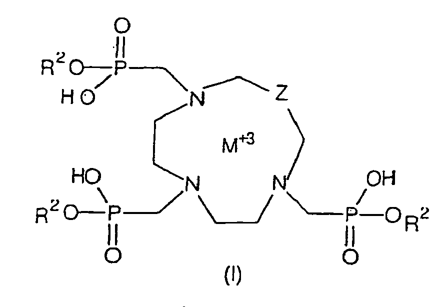 wherein Z is 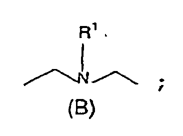 R¹ is 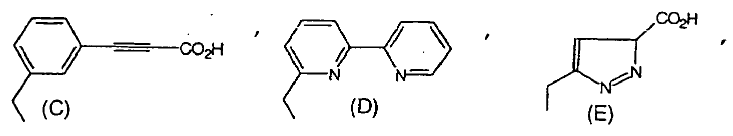 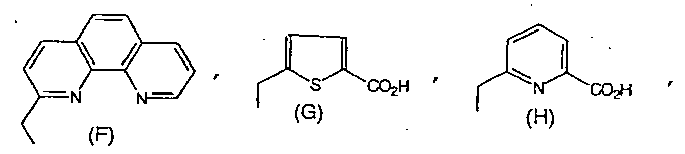 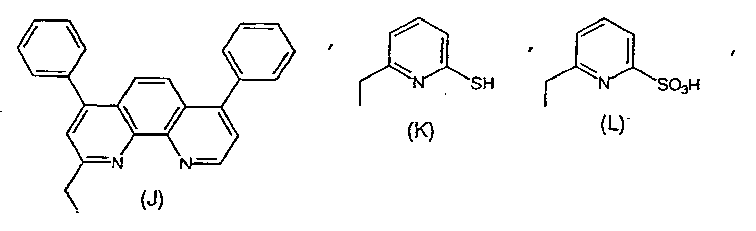 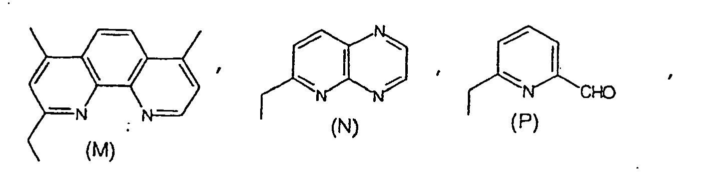 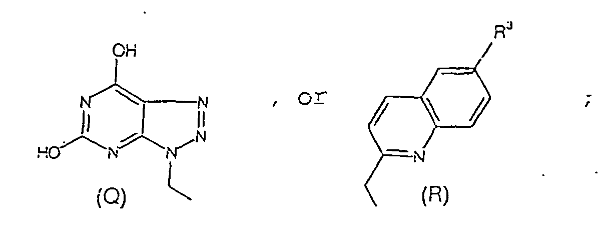 R² is methyl, ethyl, propyl, butyl or H; and
   R³ is F, C1-C4 alkyl, ,O(C₁-C₄ alkyl) or Cl;
   M is a radioactive metal ion; or
   pharmaceutically-acceptable salts thereof; and
(2) a pharmaceutically acceptable carrier for the manufacture of a medicament for the treatment of a disease state in an animal, the disease state being a soft tissue tumor or cancer.

In another embodiment, the present invention concerns a pharmaceutical formulation for the therapeutic treatment of a mammal having a disease state comprising:
(1) a radioactive chelate of formula (I) or pharmaceutically-acceptable salts thereof; and (2) a pharmaceutically acceptable carrier.

It would be advantageous to use a small molecule therapeutic agent that would localize in a specific tissue of the body without the need for attachment to a delivery molecule such as an antibody.

The medicament manufactured by the use according to this invention is for the therapeutic treatment of a mammal having a disease state. In most embodiments, the disease state will be a soft tissue tumor or cancer such as epithelial cancer or cancer of the lymphatic system. Examples of epithelial cancer include cancer of the skin, colon, oral cavity, or cervix.

The composition or medicaments manufactured by the use according to the invention have a radionuclide or metal complexed with a tetraazamacrocyclic chelating agent. As will be more fully discussed later, the properties of the radionuclide, of the chelating agent and of the complex formed therefrom are important considerations in determining the effectiveness of any particular composition or medicament for such treatment.

For the purposes of this invention, the term "tumor" shall denote a neoplasm, a new abnormal growth of tissue that is not inflammatory, which arises without obvious cause from cells of preexistent tissue, and generally possesses no physiologic function. Examples may include "carcinomas" which originate from epithelial cell, "sarcomas" of mesodermal (connective tissue) origin, and lymphomas from the lymphatic system. The origin of the neoplasm is not critical this invention.

As used herein, "complex" refers to a chelating agent complexed with a metal ion, preferably a +3 metal ion, especially a radioactive rare-earth type metal ion, wherein at least one metal atom is chelated or sequestered; "radioactive" when used in conjunction with the word "metal ion" refers to one or more isotopes of the rare-earth type elements that emit particles and/or photons. The term "radionuclide" or "metal" indicates the metal ion. When the ligand to metal ratio is discussed, the ratio is molar. The metal ligand complexes of this invention can consist of a formulation having the combination of 1 metal with 1 ligand in the form of a complex and having one or more complexes comprised of a different metal and/or different ligand, present in the same formulation. An example of this would be combining one metal ion that is gamma emitting radionuclide for imaging with a ligand and also having present another metal that is a particle emitter for the therapy with the same or different ligand. The combination of radionuclides may be more efficacious than either radionuclide alone. These combinations of complexes may be prepared by administrating two complexes at about the same time to the mammal, or making each complex separately and mixing them prior to use, or mixing the two metal ions with the same ligand and preparing the two or more complexes concurrently.

The radionuclide used in the complex of the present invention may be suitable for therapeutic purposes. Examples of the radionuclide used for therapeutic purposes are ¹⁶⁶Ho, ¹⁶⁵Dy, ⁹⁰Y, ^{115m}In, ⁵²Fe, or ⁷²Ga, ²²⁵Ac preferably ¹⁶⁶Ho, ⁹⁰Y, ¹⁵³Sm, ¹⁷⁷Lu, ¹⁷⁵Yb, ¹⁵⁹Gd, or ⁴⁷Sc.

Radionuclides can be produced in several ways. In a nuclear reactor, a nuclide is bombarded with neutrons to obtain a radionuclide, for example,

Sm-152 + neutron → Sm-153 + gamma

Another method of obtaining radionuclides is by bombarding nuclides with linear accelerator or cyclotron-produced particles. Yet another way of obtaining radionuclides is to isolate them from fission product mixtures. The method of obtaining the radionuclide is not critical to the present invention.

To irradiate Sm₂O₃ for production of Sm-153, the desired amount of target is first weighed into a quartz vial, the vial is flame sealed under vacuum and welded into aluminum can. The can is irradiated for the desired length of time, cooled for several hours and opened remotely in a hot cell. The quartz vial is removed and transferred to a glove box, crushed into a glass vial which is then sealed with a rubber septum and an aluminum crimp cap. One milliliter of 1-4 M HCl is then added to the vial via syringe to dissolve the Sm₂O₃. Once dissolved, the solution is diluted to the appropriate volume by addition of water. The solution is removed from the original dissolution vial which contains shards of the crushed quartz vial and transferred via syringe to a clean glass serum vial. This solution is then used for complex preparation. Similar procedures are used to prepare ¹⁷⁷Lu, ¹⁵⁹Gd, and ¹⁶⁶Ho. All radionuclides for this invention are either available commercially or are available from the reactor at the University of Missouri at Columbia.

When aqueous solutions of metal ions are mixed with solutions containing complexing agents, such as tetraazamacrocyclic compounds, a complex between the metal ion and the ligand can be formed as shown by the equation below.

M + L M ·L

The reaction is believed to be in equilibrium such that the concentrations of metal (M) and complexing agent, or ligand (L), can affect the concentration of species present in solution. Competing side reactions, such as metal hydroxide formation, can also occur in aqueous solution, thus

xM + yOH- → Mx(OH)y

The OH- concentration in solution, which is related to pH is, therefore, an important parameter to be considered. If the pH is too high, the metal tends to form metal hydroxides rather than complexes. The complexing agents may also be adversely affected by low pH. Complexation may require the loss of proton(s); therefore at low pH, conditions may not be favorable for complexation to occur. Consideration must be given to the solubility characteristics of the ligand, radionuclide, and complex. Although not limited thereto, a pH in the range of from 5 to 11 is preferred for complexation.

The chelating agent, or ligand, is a tetraazamacrocyclic compound having the formula: 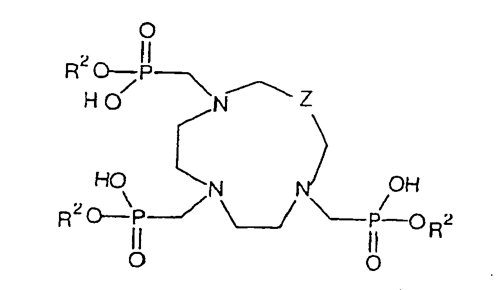 wherein Z is 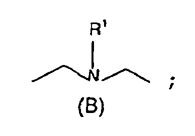 R¹ is 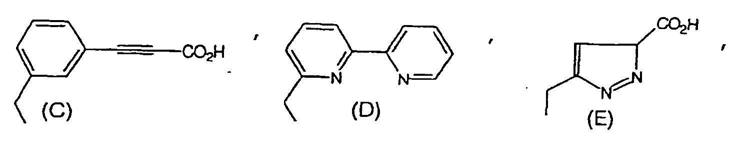 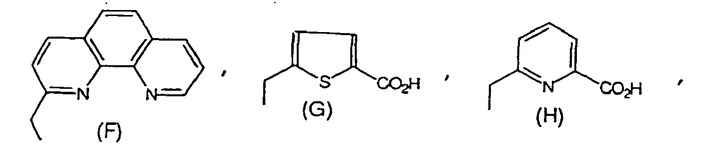 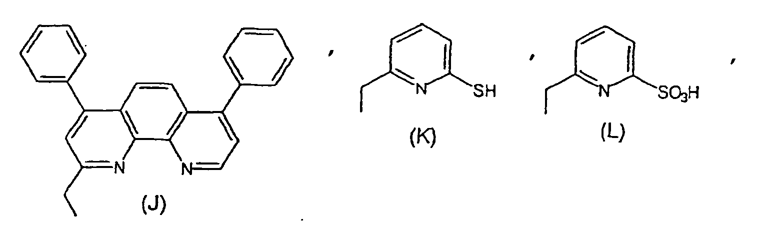 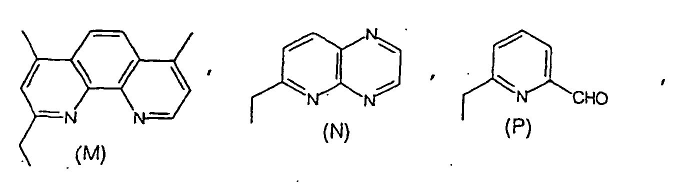 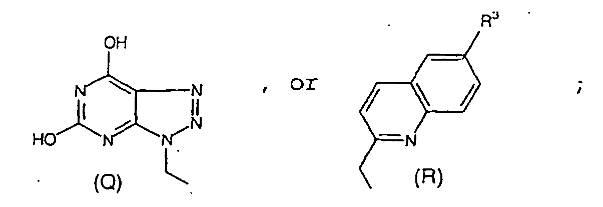 R² is methyl, ethyl, propyl, butyl or H; and
R³ is F, C1-C4 alkyl, O(C₁-C₄ alkyl) or Cl;
or a pharmaceutically acceptable salt thereof.

For the purpose of the present invention, the complexes described herein and physiologically acceptable salts thereof are considered equivalent in the therapeutically effective compositions or medicaments. Physiologically acceptable salts refer to the acid addition salts of those bases which will form a salt with at least one acid group of the ligand employed and which will not cause a significant adverse physiological effect when administered to a mammal at dosages consistent with good pharmacological practice. Suitable bases include, for example, the alkali metal and alkaline earth metal hydroxides, carbonates, and bicarbonates such as sodium hydroxide, potassium hydroxide, calcium hydroxide, potassium carbonate, sodium bicarbonate, magnesium carbonate, ammonia, primary, and secondary and tertiary amines. Physiologically acceptable salts may be prepared by treating the acid with an appropriate base.

The metal and ligand may be combined under any conditions which allow the two to form a complex. Generally, mixing in water at a controlled pH (the choice of pH is dependent upon the choice of metal) is all that is required. Most of the complexes employed in this invention were prepared as follows: the desired, amount or ligand was placed in a vial and dissolved by addition of water. The appropriate amount of the samarium, or other radionuclide, in the stock solution described above was then added to the ligand solution. The pH of the resulting solution was then adjusted to the appropriate level (usually 7-8). Additionally, the complex used in this invention may be a mixture of the different metals as described under the complex term before. In the formulation used for the manufacture of a medicament according to this invention it is necessary to employ the complex in the presence of an excess of ligand. The ligand to metal ratio (L:M) of the ligand to radionuclide or metal is at least 50:1. The upper limit of L:M depends on the toxicity of the ligand or the specific activity of the radionuclide. The preferred range for the L:M ratio is from 50:1 to 600:1, preferably from 100:1 to 500:1, especially 250:1 to 300:1.

When the radionuclide is used in the no carrier added form, then the upper L:M range could be significantly higher, such as 5X10⁷:1.

As used herein, the term "mammal" means animals that nourish their young with milk secreted by mammary glands, preferably warm blooded mammals, more preferably humans.

As used herein, "pharmaceutically acceptable salt" means any salt of the ligand which is sufficiently nontoxic to be useful in therapy or diagnosis of mammals . Thus, the salts are useful in accordance with this invention. Representative of those salts, which are formed by standard reactions, from both organic and inorganic sources include, for example, sulfuric, hydrochloric, phosphoric, acetic, succinic, citric, lactic, maleic, fumaric, palmitic, cholic, palmoic, mucic, glutamic, d-camphoric, glutaric, glycolic, phthalic, tartaric, formic, lauric, steric, salicylic, methanesulfonic, benzenesulfonic, sorbic, picric, benzoic, cinnamic acids and other suitable acids. Also included are salts formed by standard reactions from both organic and inorganic sources such as ammonium, alkali metal ions, alkaline earth metal ions, and other similar ions. Particularly preferred are the salts of the compounds of formula (I) where the salt is calcium, magnesium, potassium, sodium, ammonium, or mixtures thereof.

The formulations of the present invention are in the solid or liquid form containing the active radionuclide complexed with the ligand. These formulations may be in kit form such that the two components (that is, ligand and metal) are mixed at the appropriate time prior to use. Whether premixed or as kit, the formulations usually require a pharmaceutically acceptable carrier.

Injectable compositions of the present invention may be either in suspension or solution form. In the preparation of suitable formulations it will be recognized that, in general, the water solubility of the salt is greater than the acid form. In solution form the complex (or when desired the separate components) is dissolved in a physiologically acceptable carrier. Such carriers comprise a suitable solvent, preservatives such as benzyl alcohol, if needed, and/or buffers. Useful solvents include, for example, water, aqueous alcohols, glycols, and phosphate or carbonate esters. Such aqueous solutions contain no more than 50 percent of the organic solvent by volume.

Injectable suspensions are compositions of the present invention that require a liquid suspending medium, with or without adjuvants, as a carrier. The suspending medium can be, for example, aqueous polyvinylpyrrolidone, inert oils such as vegetable oils or highly refined mineral oils, or aqueous carboxymethylcellulose. Suitable physiologically acceptable adjuvants, if necessary to keep the complex in suspension, may be chosen from among thickeners such as carboxymethylcellulose, polyvinylpyrrolidone, gelatin, and the alginates. Many surfactants are also useful as suspending agents, for example, lecithin, alkylphenol, polyethylene oxide adducts, napthalenesulfonates, alkylbenzenesulfonates, and the olyoxyethylene sorbitan esters.

Many substances which affect the hydrophilicity, density, and surface tension of the liquid suspension medium can assist in making injectable suspension in individual cases. For example, silicone antifoams, sorbitol, and sugars are all useful suspending agents.

An "effective amount" of the formulation is used for therapy. The dose will vary depending on the disease being treated. The invention described herein provides a means of delivering a therapeutic amount of radioactivity to soft tissue tumors. However, it may also be desirable to administer a "sub-therapeutic" amount to determine the fate of the radoinuclide using a scintillation camera prior to administering a therapeutic dose or if diagnostic images are the desired result. Therapeutic doses will be administered in sufficient amounts to reduce pain and/or inhibit tumor growth and/or cause regression of tumors and/or kill the tumor. Amounts of radionuclide needed to provide the desired therapeutic dose will be determined experimentally and optimized for each particular composition. The amount of radioactivity required to deliver a therapeutic dose will vary with the individual composition employed. The composition or medicament to be administered may be given in a single treatment or fractionated into several portions and composition in fractionated doses may make it possible to minimize damage to non-target tissue. Such multiple dose administration may be more effective.

The compositions or medicaments of the present invention may be used in conjunction with other active agents and/or ingredients that enhance the therapeutic effectiveness of the compositions and/or facilitate easier administration of the compositions.

While not wishing to be bound by theory, it is believed that the advantageous results of the present invention are obtained because of the possible uptake preferentially in the tumor. The mechanism of uptake of the radionuclide by neoplastic tissue is not clear. Some suggested mechanisms are:
a) An imbalance between arterial blood supply to the tumor and venous drainage from the tumor. A reduced venous drainage would result in an increase in concentration of the material within the tumor mass.
b) Lymphatic drainage from a tumor may be decreased.
c) Non-specific binding to protein within the tumor may occur.
d) Because inflammatory reaction is usually present near a tumor, this may result in the differential concentration of radiolabel within the tumor.
e) MetallothionEin a protein binder of heavy metals.
f) Several mechanisms may be involved.

Although the theory for the mechanism of action is still unknown, the present invention provides a complex which allows metal ions to locate in the tumor and displays low uptake in other tissues, for example, liver.

The following definitions are provided for some terms that are used throughout this text.

### Glossary:

Conc. = concentrated
mG = milligrams
mCi = milliCuries
HEDTA = Hydroxyethylethylenediaminetriacetic acid
Ac = Actinium
Sm = Samarium
Ho = Holmium
Yb = Ytterbium
Y = Yttrium
Gd = Gadolinium
Lu = Lutetium
In = Indium
Sc = Scandium
Fe = iron
Ga = Gallium
chelant is equivalent to ligand
complex is equivalent to chelate, and
L:M = ligand to metal molar ratio.

The invention will be further clarified by a consideration of the following examples, which are intended to be purely exemplary of the present invention.

### Biodistribution Studies

### Example 1

The details of the tissue biodistribution studies are as follows. A ¹⁷⁷LuCl₃ solution was prepared as were the appropriate ligand Biodistribution solutions. The two solutions were thoroughly mixed at a pH = 2 and the pH of the solution was raised to 7 using 0.1N NaOH to facilitate complexation. Complexation was then evaluated by passing the sample solution (100 µL) through a Sephadex^{™} C-25 column eluting (2 x 3 mL) with 4:1 saline (0.85 percent NaCl/NH₄H) and comparing the amount of radioactivity in the eluent with that remaining on the column (free metal remains on the column). The in vivo distribution of the radioactive complexes was measured using three Sprague Dawley rats (180-220 g), each injected with 100 µL (pH = 7.5) of the radioactive complex solution. After 30 minutes or 2 hours, the animals were sacrificed. The organs were removed, weighed and counted. The total percentage of the dose in bone was calculated using the standard assumptions regarding the total body weight percentages. For example, the bone sample (femur) represents 1/25 the weight of the total skeletal system and total muscle dose was calculated by assuming that muscle comprises 43 percent of the total body weight. 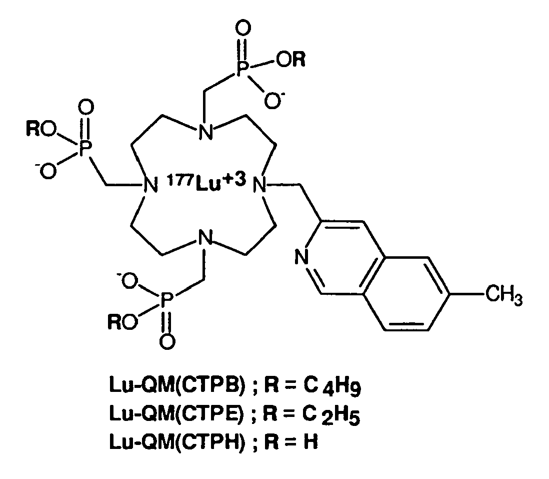 Butyl-ester-quinolin, Lu-177 [Lu-QM(CTPB)
10:1, Ligand:Metal Molar Ratio, Ligand = 2.0 mM

| PERCENT DOSE | | | | | |
|---|---|---|---|---|---|
| | RAT 1 | RAT 2 | RAT 3 | AVERAGE | +/- |
| BONE | 10.34 | 12.32 | 10.22 | 10.96 | 1.18 |
| TAIL | 6.43 | 2.38 | 8.59 | 5.80 | 3.15 |
| LIVER | 10.85 | 11.29 | 9.62 | 10.59 | 0.86 |
| KIDNEY | 3.18 | 3.96 | 3.39 | 3.51 | 0.40 |
| SPLEEN | 0.60 | 0.54 | 0.67 | 0.60 | 0.06 |
| MUSCLE | 4.93 | 4.42 | 4.47 | 4.61 | 0.28 |
| BLOOD | 7.11 | 6.67 | 6.05 | 6.61 | 0.54 |
| HEART | 0.10 | 0.14 | 0.14 | 0.13 | 0.02 |
| LUNG | 0.43 | 0.30 | 0.74 | 0.49 | 0.22 |
| BRAIN | 0.02 | 0.03 | 0.16 | 0.07 | 0.08 |
| STOMACH | 0.09 | 1.32 | 2.22 | 1.21 | 1.07 |
| SMALL INT1 | 2.28 | 1.49 | 40.60 | 14.79 | 22.36 |
| SMALL INT2 | 40.88 | 43.37 | 1.70 | 28.65 | 23.37 |
| CEACUM | 0.59 | 0.23 | 0.11 | 0.31 | 0.25 |
| COLON | 0.10 | 0.12 | 0.25 | 0.16 | 0.08 |
| TESTES | 0.29 | 0.33 | 0.19 | 0.27 | 0.07 |
| PANCREAS | 0.17 | 0.15 | 0.00 | 0.11 | 0.09 |
| URINE | 13.26 | 10.89 | 12.51 | 12.22 | 1.21 |

| TOTAL ACCOUNTABILITY | | | | | |
|---|---|---|---|---|---|
| RAT 1 | RAT 2 | RAT 3 | | AVERAGE | +/- |
| 101.67 | 99.94 | | 101.60 | 101.07 | 0.69 |

DATE LIGAND
1-30-01, Ethyl-ester-quinolin, Lu-177
Lu-QM(CTPE)
10:1, Ligand:Metal Molar Ratio, Ligand = 2.0 mM

| | RAT 1 | RAT 2 | +/- |
|---|---|---|---|
| BONE | 7.95 | 9.36 | 0.87 |
| TAIL | 6.49 | 0.94 | 2.81 |
| LIVER | 2.87 | 4.29 | 4.47 |
| KIDNEY | 3.98 | 3.79 | 0.62 |
| SPLEEN | 0.20 | 1.10 | 0.56 |
| MUSCLE | 3.86 | 4.31 | 0.80 |
| BLOOD | 4.13 | 6.37 | 1.59 |
| HEART | 0.12 | 0.11 | 0.04 |
| LUNG | 0.24 | 0.49 | 0.16 |
| BRAIN | 0.01 | 0.00 | 0.01 |
| STOMACH | 0.23 | 11.76 | 6.14 |
| SMALL INT1 | 0.61 | 0.98 | 1.64 |
| SMALL INT2 | 2.76 | 14.02 | 5.72 |
| CEACUM | 4.02 | 3.75 | 2.17 |
| COLON | 0.16 | 0.10 | 0.04 |
| PANCREAS | 0.05 | 0.07 | 0.01 |
| URINE | 9.60 | 79.72 | 35.53 |

| | TOTAL ACCOUNTABILITY | |
|---|---|---|
| RAT 1 | RAT 2 | +/- |
| 47.27 | 141.17 | 33.74 |

DATE LIGAND
1-30-01, Phosphonic acid-quinolin, Lu-177 Lu-QM(CTPH)
10:1, Ligand:Metal Molar Ratio, Ligand = 2.0 mM

| | RAT 1 | RAT 2 | +/- |
|---|---|---|---|
| BONE | 44.87 | 26.77 | 9.07 |
| TAIL | 2.35 | 10.86 | 4.78 |
| LIVER | 5.81 | 3.62 | 1.68 |
| KIDNEY | 2.29 | 3.12 | 0.52 |
| SPLEEN | 0.49 | 0.12 | 0.23 |
| MUSCLE | 12.65 | 10.20 | 1.23 |
| BLOOD | 16.95 | 12.50 | 2.31 |
| HEART | 0.53 | 0.32 | 0.12 |
| LUNG | 1.26 | 0.76 | 0.37 |
| BRAIN | 0.09 | 0.08 | 0.02 |
| STOMACH | 0.71 | 6.00 | 2.75 |
| SMALL INT1 | 1.21 | 5.32 | 2.06 |
| SMALL INT2 | 1.07 | 1.89 | 1.95 |
| CEACUM | 0.28 | 0.17 | 0.09 |
| COLON | 0.20 | 0.12 | 0.09 |
| PANCREAS | 0.12 | 0.08 | 0.09 |
| URINE | 16.20 | 14.00 | 6.07 |

| TOTAL ACCOUNTABILITY | | |
|---|---|---|
| RAT 1 | RAT 2 | +/- |
| 107.10 | 95.92 | 4.69 |

### Example 2

Biodistribution studies were carried out as described above using the corresponding ¹⁵³Sm complexes. Approximately 1 mg/kg of solute was administered to the rat. After 2 hours, the animals were euthanized and their tissues removed, weighed and counted for radioactivity. The total percentage of the dose in bone was calculated by assuming that the bone sample (femur) represents 1/25th of the total weight of the skeletal system and distribution is uniform. The total blood dose was calculated by assuming that the blood comprises 6.5 percent of the total body weight. The total muscle dose was calculated by assuming that the muscle comprises 43 percent of the total body weight.

## Claims

1. A pharmaceutical formulation for the therapeutic treatment of a mammal having- a disease state comprising-:
(1) a radioactive chelate having a formula: 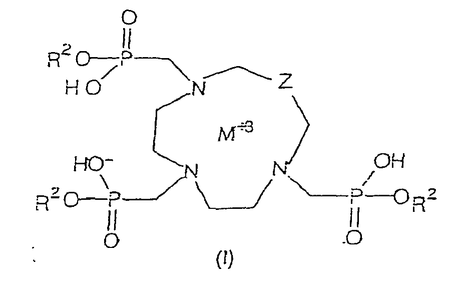 wherein Z is 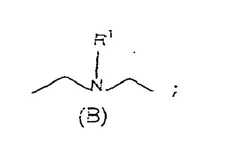 R¹ is 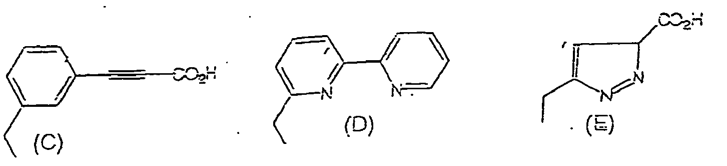 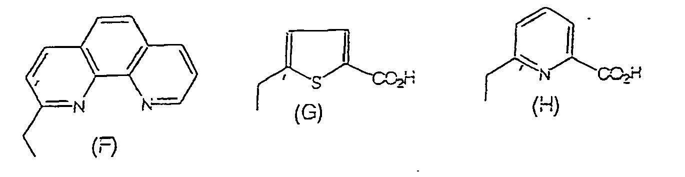 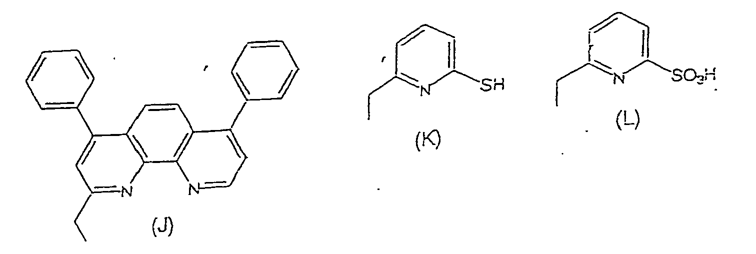 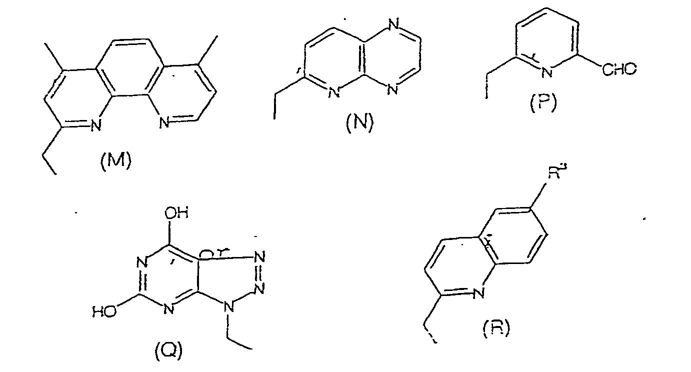 R² is methyl, ethyl, propyl, butyl or H; and
R³ is F, C1-C4 alkyl, O(C₁-C₄ alkyl) or Cl;
M is a radioactive metal ion; or
pharmaceutically-acceptable salts thereof ; and
(2) a pharmaceutically acceptable carrier.

2. The formulation of Claim 1 wherein M is ¹⁵³Sm ¹⁶⁶Ho, ⁹⁰Y, ¹⁶⁵Dy, ²⁵⁹Gd, ¹⁷⁷Lu ¹¹¹In, ^{115m}In, ¹⁷⁵Yb, ²⁷Sc, ⁵³Fe, ⁷²Ga, ⁶⁷Ga, ⁶⁸Ga, ²²⁵Ac, or Fe.

3. The formulation of Claim 1 wherein the chelate has a ligand to metal molar ratio of at least 50:1.

4. The formulation of claim 1 where the formulation is in a form to be administered topically or as an injectable solution.

5. The formulation of claim 4 wherein for topical applications the chelate is formulated at a concentration of 1 ·M-1-10mM in an aqueous solution.

6. The formulation of claim 4 wherein for injectable application the chelate is to be administered at 0.001-0.2 mmol/Kg of body weight.

7. The formulation of claim 1 wherein the disease state is epithelial cancer or cancer of the lymphatic system.

8. The formulation of claim 7 wherein the epithelial cancer is in the skin, colon, oral cavity, or cervix.

9. Use of a formulation comprising a radioactive chelate having a formula: 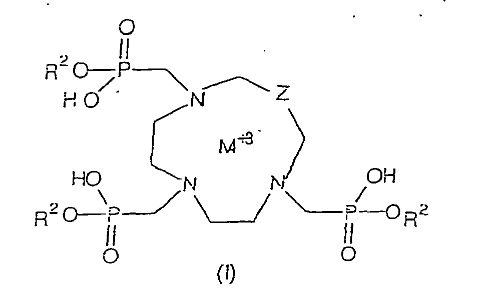 wherein Z is 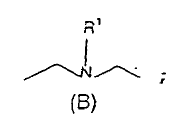 R¹ is 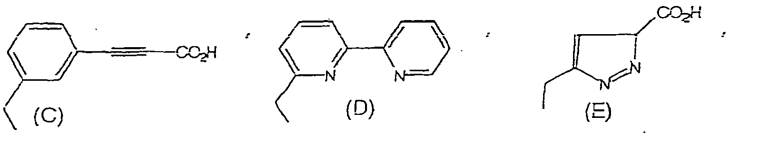 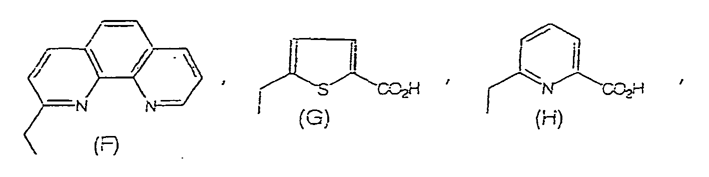 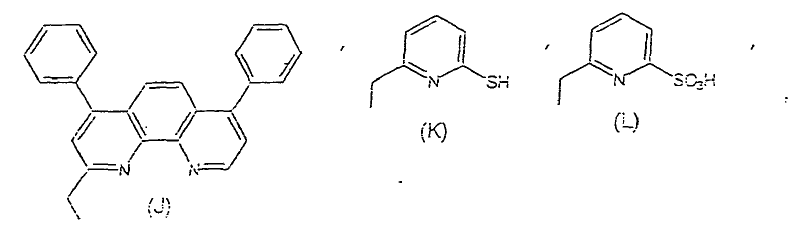 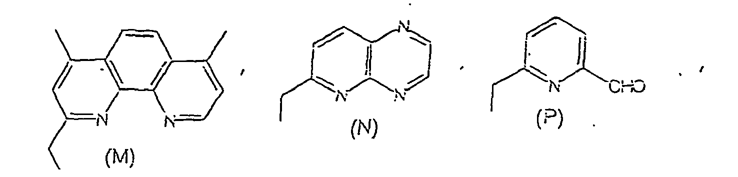 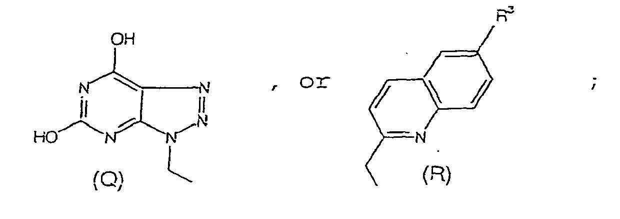 R² is methyl, ethyl, propyl, butyl or H; and
R³ is F, C1-C4 alkyl, O(C₁-C₄ alkyl) or Cl;
M is a radioactive metal ion; or
pharmaceutically-acceptable salts thereof; and
a pharmaceutically acceptable carrier
for the manufacture of a medicament for the treatment of a disease state selected from soft tissue tumor or cancer.

10. The use of Claim 9 wherein M is ¹⁵³Sm ¹⁶⁶Ho, ⁹⁰Y, ¹⁶⁵Dy, ¹⁵⁹Gd, ¹⁷⁷Lu, ¹¹¹In, ^{115m}In ¹⁷⁵Yb, ⁶⁷Sc, ⁵²Fe, ⁷²Ga, ⁶⁷Ga, ⁶⁸Ga, ²²⁵Ac, or Fe.

11. The use of Claim 9 wherein the chelate has a ligand to metal molar ratio of at least 50:1.

12. The use of claim 9 where the formulation is in a form to be administered topically or as an injectable solution.

13. The use of claim 12 wherein for topical applications the chelate is formulated at a concentration of 1 ·M-10mM in an aqueous solution.

14. The use of claim 12 wherein for injectable application the chelate is to be administered at 0.001-0.2 mmol/Kg of body weight.

15. The use of claim 9 wherein the disease state is epithelial cancer or cancer of the lymphatic system.

16. The use of claim 15 wherein the epithelial cancer is in the skin, colon, oral cavity, or cervix.

## Patentansprüche

1. Pharmazeutische Formulierung zur therapeutischen Behandlung eines einen Krankheitszustand aufweisenden Säugers, umfassend:
(1) ein radioaktives Chelat mit einer Formel: 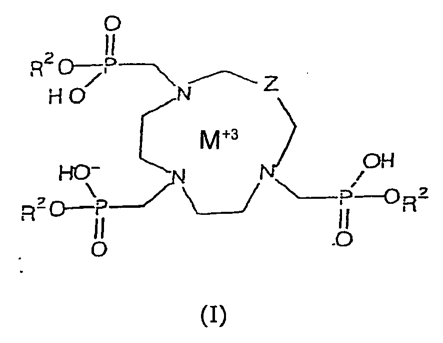 wobei Z 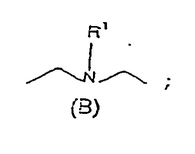 ist;
R¹ 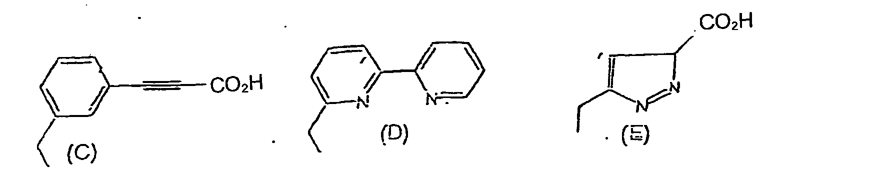 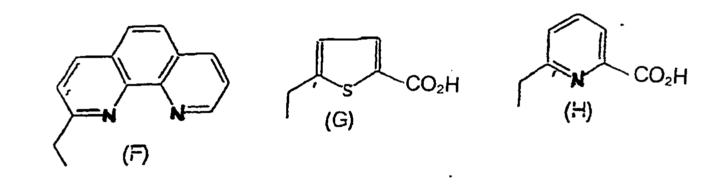 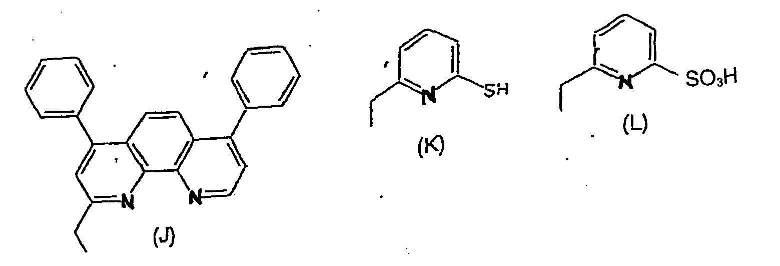 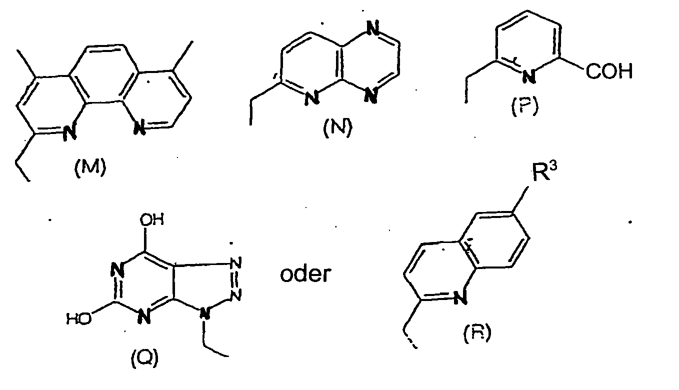 ist;
R² Methyl, Ethyl, Propyl, Butyl oder H ist; und
R³ F, C₁-C₄ Alkyl, O(C₁-C₄ Alkyl) oder Cl ist;
M ein Ion eines radioaktiven Metalls ist; oder pharmazeutisch-annehmbare Salze davon; und
(2) einen pharmazeutisch annehmbaren Träger.

2. Formulierung nach Anspruch 1, wobei M ¹⁵³Sm, ¹⁶⁶Ho, ⁹⁰Y, ¹⁶⁵Dy, ¹⁵⁹Gd, ¹⁷⁷Lu, ¹¹¹In, ^{115m}In, ¹⁷⁵Yb, ⁴⁷SC ⁵²Fe, ⁷²Ga, ⁶⁷Ga, ⁶⁸Ga, ²²⁵Ac oder Fe ist.

3. Formulierung nach Anspruch 1, wobei das Chelat ein molares Verhältnis von Ligand zu Metall von wenigstens 50:1 aufweist.

4. Formulierung nach Anspruch 1, wobei die Formulierung in einer topischen Verabreichungsform oder als eine injizierbare Lösung vorliegt.

5. Formulierung nach Anspruch 4, wobei das Chelat für topische Anwendungen mit einer Konzentration von 1 • M - 10mM in einer wässrigen Lösung formuliert ist.

6. Formulierung nach Anspruch 4, wobei das Chelat bei einer injizierbaren Anwendung mit 0,001-0,2 mmol/kg Körpergewicht zu verabreichen ist.

7. Formulierung nach Anspruch 1, wobei der Krankheitszustand Epithelkrebs oder eine Krebserkrankung des Lymphgefäßsystems ist.

8. Formulierung nach Anspruch 7, wobei der Epithelkrebs in der Haut, dem Dickdarm, der Mundhöhle oder dem Hals vorliegt.

9. Verwendung einer Formulierung, umfassend ein radioaktives Chelat mit einer Formel: 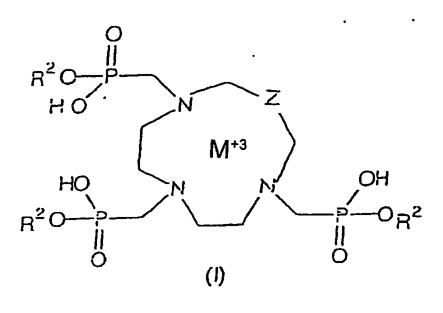 wobei Z 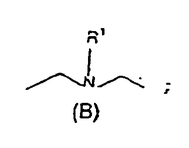 ist;
R¹ 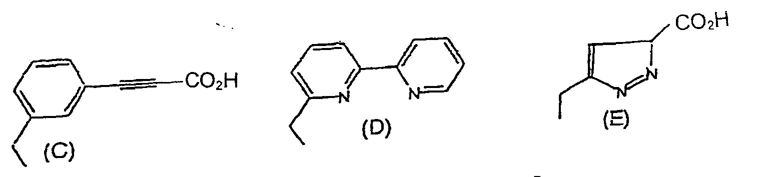 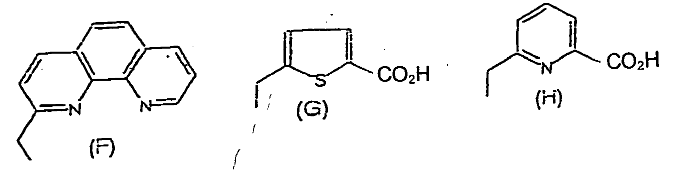 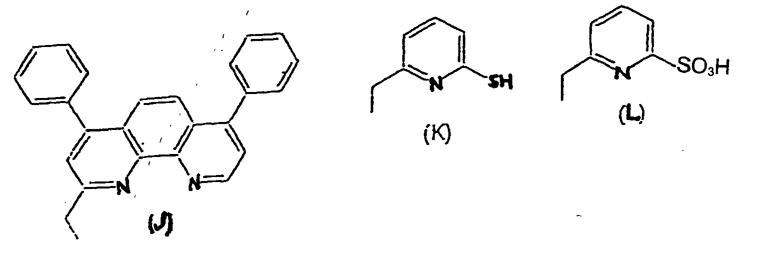 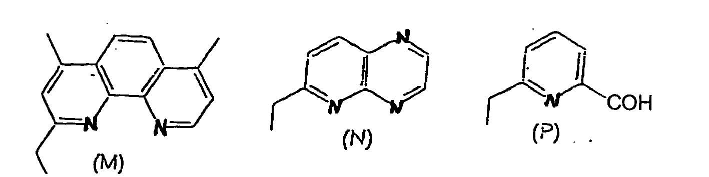 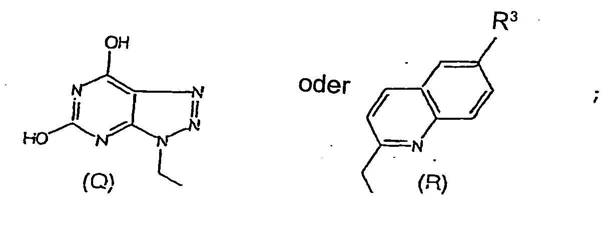 ist;
R² Methyl, Ethyl, Propyl, Butyl oder H ist; und
R³ F, C₁-C₄ Alkyl, O(C₁-C₄ Alkyl) oder Cl ist;
M ein Ion eines radioaktiven Metalls ist; oder pharmazeutisch-annehmbare Salze davon; und einen pharmazeutisch annehmbaren Träger
zur Herstellung eines Medikaments zur Behandlung eines Krankheitszustands ausgewählt aus Weichgewebetumor oder Krebs.

10. Verwendung nach Anspruch 9, wobei M ¹⁵³Sm, ¹⁶⁶Ho, ⁹⁰Y, ¹⁶⁵Dy, ¹⁵⁹Gd, ¹⁷⁷Lu, ¹¹¹In, ^{115m}In, ¹⁷⁵Yb, ⁴⁷Sc, ⁵²Fe , ⁷²Ga , ⁶⁷Ga , ⁶⁸Ga , ²²⁵Ac oder Fe ist.

11. Verwendung nach Anspruch 9, wobei das Chelat ein molares Verhältnis von Ligand zu Metall von wenigstens 50:1 aufweist.

12. Verwendung nach Anspruch 9, wobei die Formulierung in einer topischen Verabreichungsform oder als eine injizierbare Lösung vorliegt.

13. Verwendung nach Anspruch 12, wobei das Chelat für topische Anwendungen mit einer Konzentration von 1 • M - 10mM in einer wässrigen Lösung formuliert ist.

14. Verwendung nach Anspruch 12, wobei das Chelat bei einer injizierbaren Anwendung mit 0,001-0,2 mmol/kg Körpergewicht zu verabreichen ist.

15. Verwendung nach Anspruch 9, wobei der Krankheitszustand Epithelkrebs oder eine Krebserkrankung des Lymphgefäßsystems ist.

16. Verwendung nach Anspruch 15, wobei der Epithelkrebs in der Haut, dem Dickdarm, der Mundhöhle oder dem Hals vorliegt.

## Revendications

1. Formulation pharmaceutique pour le traitement thérapeutique d'un mammifère ayant un état maladif comprenant :
(1) un chélate radioactif ayant une formule : 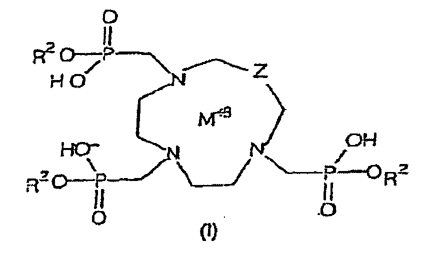 dans laquelle Z est 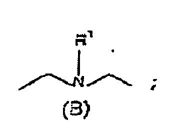 R¹ est 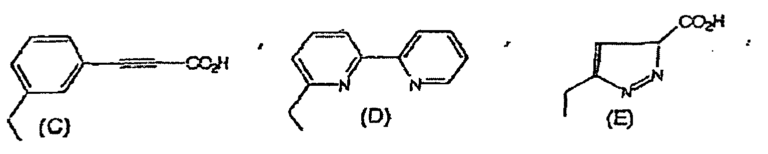 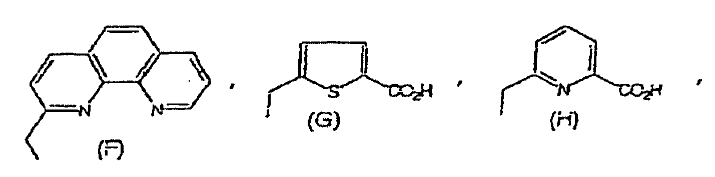 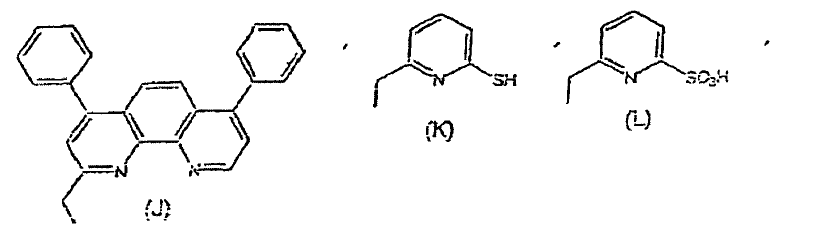 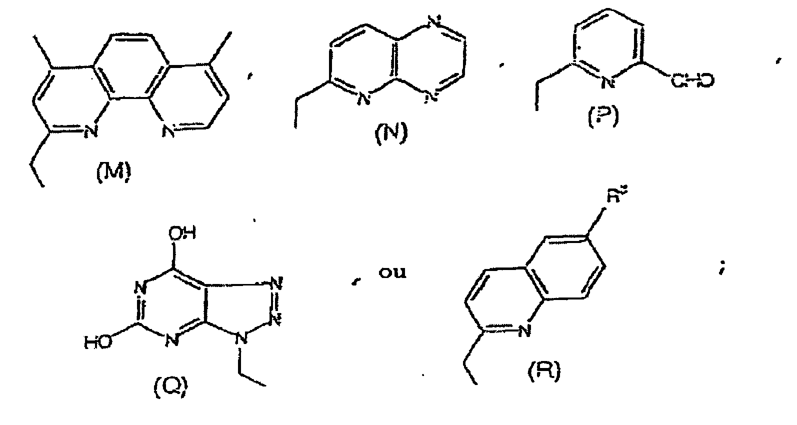 R² est un groupe méthyle, éthyle, propyle, butyle ou H ; et
R³ représente F, alkyle en C₁-C₄, O- (alkyle en C₁-C₄) ou Cl ;
M est un ion métallique radioactif ; ou un de ses sels pharmaceutiquement acceptables ; et
(2) un support pharmaceutiquement acceptable.

2. Formulation selon la revendication 1, dans laquelle M est ¹⁵³Sm, ¹⁶⁶Ho, ⁹⁰Y, ¹⁶⁵Dy, ¹⁵⁹Gd, ¹⁷⁷Lu, ¹¹¹In, ^{115m}In, ¹⁷⁵Yb, ⁴⁷Sc, ⁵²Fe, ⁷²Ga, ⁶⁷Ga, ⁶⁸Ga, ²²⁵Ac, ou Fe.

3. Formulation selon la revendication 1, dans laquelle le chélate possède un rapport molaire entre ligand et métal d'au moins 50/1.

4. Formulation selon la revendication 1, dans laquelle la formulation est sous une forme devant être administrée par voie topique ou en tant que solution injectable.

5. Formulation selon la revendication 4, dans laquelle le chélate est formulé pour des applications par voie topique à une concentration de 1 mM à 10 mM dans une solution aqueuse.

6. Formulation selon la revendication 4, dans laquelle le chélate doit être administré à 0,001 à 0,2 mmol/kg de poids corporel pour une application injectable.

7. Formulation selon la revendication 1, dans laquelle l'état maladif est un cancer épithélial ou cancer du système lymphatique.

8. Formulation selon la revendication 7, dans laquelle le cancer épithélial est dans la peau, le colon, la cavité buccale ou le col de l'utérus.

9. Utilisation d'une formulation comprenant un chélate radioactif ayant une formule : 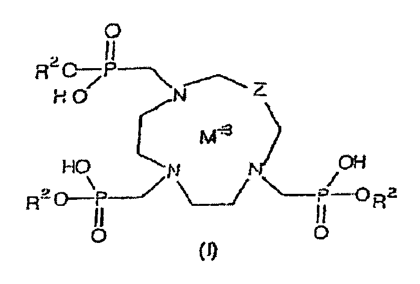 dans laquelle Z est 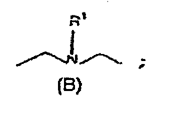 R¹ est 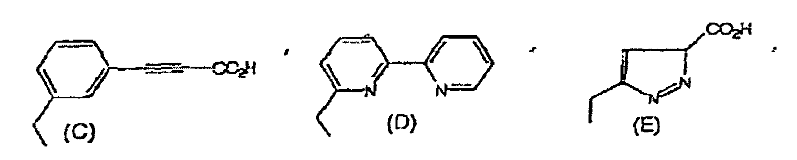 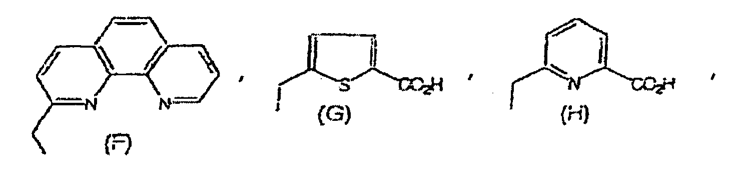 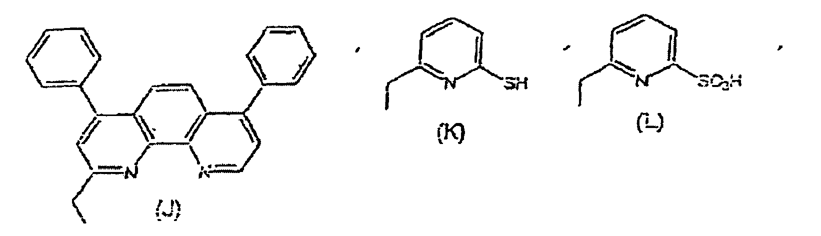 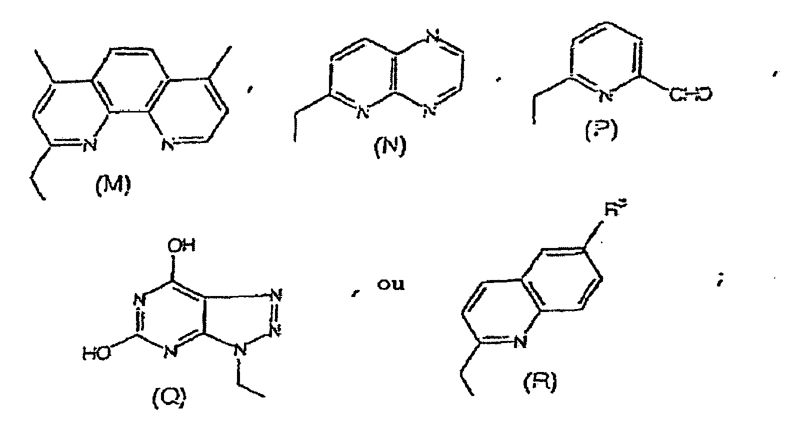 R² est un groupe méthyle, éthyle, propyle, butyle ou H ; et
R³ représente F, alkyle en C₁-C₄, O- (alkyle en C₁-C₄) ou Cl ;
M est un ion métallique radioactif ; ou
un de ses sels pharmaceutiquement acceptables ; et
un support pharmaceutiquement acceptable pour la fabrication d'un médicament pour le traitement d'un état maladif sélectionné parmi une tumeur ou un cancer des parties molles.

10. Utilisation selon la revendication 9, dans laquelle M est ¹⁵³Sm, ¹⁶⁶Ho, ⁹⁰Y, ¹⁶⁵Dy, ¹⁵⁹Gd, ¹⁷⁷Lu, ¹¹¹In, ^{115m}In, ¹⁷⁵Yb, ⁴⁷Sc, ⁵²Fe, ⁷²Ga,⁶⁷Ga, ⁶⁸Ga, ²²⁵Ac, ou Fe.

11. Utilisation selon la revendication 9, dans laquelle le chélate possède un rapport molaire entre ligand et métal d'au moins 50/1.

12. Utilisation selon la revendication 9, dans laquelle la formulation est sous une forme devant être administrée par voie topique ou en tant que solution injectable.

13. Utilisation selon la revendication 12, dans laquelle le chélate est formulé pour des applications par voie topique à une concentration de 1 mM à 10 mM dans une solution aqueuse.

14. Utilisation selon la revendication 12, dans laquelle le chélate doit être administré à 0,001 à 0,2 mmol/kg de poids corporel pour une application injectable.

15. Utilisation selon la revendication 9, dans laquelle l'état maladif est un cancer épithélial ou cancer du système lymphatique.

16. Utilisation selon la revendication 15, dans laquelle le cancer épithélial est dans la peau, le colon, la cavité buccale ou le col de l'utérus.
